(19) Europäisches Patentamt
European Patent Office
Office européen des brevets

(11) **EP 1 980 271 A1**

(12) **EUROPEAN PATENT APPLICATION**
published in accordance with Art. 158(3) EPC

(43) Date of publication:
**15.10.2008 Bulletin 2008/42**

(51) Int Cl.:
*A61K 45/00* (2006.01)          *A61K 9/08* (2006.01)
*A61K 9/10* (2006.01)          *A61K 9/107* (2006.01)
*A61K 9/12* (2006.01)          *A61K 9/14* (2006.01)
*A61K 31/165* (2006.01)          *A61K 47/14* (2006.01)
*A61K 47/18* (2006.01)          *A61P 25/24* (2006.01)
*A61P 29/00* (2006.01)

(21) Application number: 07707473.0

(22) Date of filing: 26.01.2007

(86) International application number:
**PCT/JP2007/051241**

(87) International publication number:
**WO 2007/086493 (02.08.2007 Gazette 2007/31)**

(84) Designated Contracting States:
**AT BE BG CH CY CZ DE DK EE ES FI FR GB GR
HU IE IS IT LI LT LU LV MC NL PL PT RO SE SI
SK TR**

(30) Priority: 27.01.2006 JP 2006019443

(71) Applicant: **Asahi Kasei Pharma Corporation
Tokyo 101-8101 (JP)**

(72) Inventor: **NATSUME, Hideshi
Kawagoe-shi
Saitama 350-1108 (JP)**

(74) Representative: **Forstmeyer, Dietmar et al
BOETERS & LIECK
Oberanger 32
80331 München (DE)**

(54) **MEDICINE FOR TRANSNASAL ADMINISTRATION**

(57)     The object is to provide an SNRI-containing preparation which has a higher absorbability compared to a conventional SNRI preparation, can produce its effect rapidly, and can be readily administered to a patient who is hard to be administered via an oral route. Disclosed is an SNRI-containing preparation for transmucosal administration or transdermal administration.

**Description**

Technical Field

The present invention relates to a preparation containing a selective serotonin/noradrenaline reuptake inhibitor as an active ingredient. More preferably, the present invention relates to a preparation containing milnacipran or a salt thereof.

Background Art

A selective serotonin/noradrenaline reuptake inhibitor (hereinafter, abbreviated as SNRI, in some cases) is the fourth-generation antidepressant, which is widely known as a medicine that selectively binds to reuptake sites of serotonin and noradrenaline, serving as neurotransmitters, in the brain-nerve connection and inhibits the uptake of them to express its antidepressant effect. Milnacipran (cis-($\pm$)-2-(aminomethyl)-N,N-diethyl-1-phenyl-cyclopropanecarboxyamide), one of typical SNRIs, inhibits reuptake of noradrenaline in addition to serotonin, and milnacipran has been developed as a novel antidepressant referred to as SNRI (Selective Serotonin/Noradrenaline Reuptake Inhibitor), which has an effect comparable to that of a tricyclic antidepressant such as imipramine and has few side effects typical of the tricyclic antidepressant (anticholinergic effect, effect on heart/circulatory organs) due to very low affinity to receptors of various brain neurotransmitters. In addition, the milnacipran and is sold as milnacipran hydrochloride, which is manufactured as a film-coating tablet (product name: Toledomin) in Japan or as a capsule (IXEL) in foreign countries. The production methods for milnacipran have been reported in Patent Documents 1 to 3. Meanwhile, milnacipran-containing preparations have been reported in Patent Documents 4 and 5.
Currently, a study has been made on the effectiveness/safety of high-dose administration of milnacipran hydrochloride for treatment of a disease to be treated with milnacipran, such as a depressive symptom, and the probability of high-dose administration has been increased (Non-patent Document 1). In the case of high-dose administration, it is necessary to take a plurality of tablets or a large tablet containing a high-dose component or to increase the number of doses, so a patient may bear a huge burden and have difficulty in taking the medicine via an oral route.

    Patent Document 1: JP 63-23186 B
    Patent Document 2: JP 05-67136 B
    Patent Document 3: JP 2964041 B
    Patent Document 4: JP 2000-516946 A
    Patent Document 5: JP 2002-519370 A
    Non-patent Document 1: Psychopharmacology vol. 5 No. , 2002, p93-99

Disclosure of the Invention

Problems to be solved by the Invention

An object of the present invention is to provide an SNRI-containing preparation, which has a higher therapeutic effect and absorbability, can express its effect rapidly, and can be readily administered compared to a conventional SNRI preparation for oral administration. In particular, an object of the present invention is to provide a milnacipran-containing preparation, which has a higher therapeutic effect and absorbability, can express its effect rapidly, and can be readily administered to a patient who is hard to be administered via an oral route compared to a conventional milnacipran preparation.

Means for solving the Problems

The inventors of the present invention have made extensive studies to solve the above-mentioned problems, and as a result, they have found that transnasal administration of milnacipran (typical SNRI) or a salt thereof can achieve efficient absorption of milnacipran, resulting in release of milnacipran in a very short time compared to oral administration. They have further found that transnasal administration of milnacipran or a salt thereof can efficiently penetration milnacipran to the central nerve system, can produce its effect rapidly compared to oral administration, and can provide its effect rapidly even at a lower dose compared to oral administration, thus completing the present invention.
That is, the present invention is as follows:

    [A1] a preparation for transmucosal administration including a selective serotonin/noradrenaline reuptake inhibitor;
    [A1-2] a preparation according to the item [A1], in which the preparation for transmucosal administration is a preparation for transnasal administration;

[A2] a preparation described in the item [A1] or [A1-2], in which the selective serotonin/noradrenaline reuptake inhibitor is milnacipran or a salt thereof;

[A3] a preparation according to the item [A2], in which milnacipran or a salt thereof is milnacipran hydrochloride;

[A4] a preparation described in any one of the items [A1] to [A3], in which the preparation is a solution;

Note that in the case where the item numbers cited are within a certain range (for example, [A1] to [A3] above), which includes an item with a sub-number such as [A1-2], the item with a sub number such as [A1-2] is cited. The same applies to the following items.

[A5] a preparation according to any one of the items [A1] to [A3], in which the preparation is a solution;

[A6] a preparation according to any one of the items [A1] to [A3], in which the preparation is an emulsion;

[A7] a preparation according to any one of the items [A4] to [A6], in which an osmotic pressure ratio of the solution, the suspension, or the emulsion is 0.5 to 5.0;

[A7-2] a preparation according to any one of the items [A4] to [A6], in which an osmotic pressure ratio of the solution, the suspension, or the emulsion is 0.5 to 2.5;

[A7-3] a preparation according to any one of the items [A4] to [A6], in which an osmotic pressure ratio of the solution, the suspension, or the emulsion is 0.5 to 1.5;

[A7-4] a preparation according to any one of the items [A4] to [A6], characterized in that the osmotic pressure ratio of the solution, the suspension, or the emulsion is about 1 or less;

[A8] a preparation according to any one of the items [A1] to [A7-4], characterized in that the preparation is an aerosol;

[A9] a preparation according to any one of the items [A1] to [A3], characterized in that the preparation is a powder;

[A10] a preparation according to any one of the items [A1] to [A9], including one or more preservatives selected from quaternary ammonium salts and parabens;

[A10-2] a preparation according to any one of the items [A1] to [A9], including one or more preservatives selected from quaternary ammonium salts;

[A11] a preparation according to any one of the items [A1] to [A10-2], characterized in that the bioavailability is 20% or more;

[A11-2] a preparation according to any one of the items [A1] to [A10-2], characterized in that the bioavailability is 60% or more;

[A11-3] a preparation according to any one of the items [A1] to [A 10-2], characterized in that the bioavailability is 80% or more;

[A11-4] a preparation according to any one of the items [A1] to [A10-2], characterized in that the bioavailability is 90% or more;

[A12] a preparation according to any one of the items [A1] to [A11-4], characterized in that the time-to-maximum blood concentration is 60 minutes or less;

[A13] a preparation according to any one of the items [A1] to [A12], which is used for a patient who is hard to be administered via an oral route;

[A14] a preparation according to any one of the items [A1] to [A13], in which the preparation is an antidepressant;

[A15] a preparation according to any one of the items [A1] to [A13], in which the preparation is an analgetic;

[A16] a preparation according to any one of the items [A1] to [A15], in which the daily dosage of milnacipran or a salt thereof, serving as an active ingredient, is 1 mg or more;

[A16-2] a preparation according to any one of the items [A1] to [A15], in which the daily dosage of milnacipran or a salt thereof, serving as an active ingredient, is 20 mg or more;

[A16-3] a preparation according to any one of the items [A1] to [A15], in which the daily dosage of milnacipran or a salt thereof, serving as an active ingredient, is 50 mg or more;

[A17] a method of transmucosally administering a selective serotonin/noradrenaline reuptake inhibitor;

[A 17-2] a method of transmucosally administering a selective serotonin/noradrenaline reuptake inhibitor;

[A17-3] a method of administering a selective serotonin/noradrenaline reuptake inhibitor by nasal drop;

[A17-4] a method according to the items [A17] or [A17-3], in which the selective serotonin/noradrenaline reuptake inhibitor is milnacipran or a salt thereof;

[A17-5] a method according to the item [A17-4], in which milnacipran or a salt thereof is milnacipran hydrochloride;

[A17-6] a method according to any one of the items [A17] to [A17-5], which has a characteristic according to any one of the items [A2] to [A16-3];

[A18] a use of milnacipran or a salt thereof for manufacturing a preparation for transmucosal administration or a preparation for transdermal administration;

[A19] a preparation for transnasal administration, which contains an antidepressant;

[A20] a preparation for transdermal administration including a selective serotonin/noradrenaline reuptake inhibitor;

[A20-2] a preparation according to the item [A20], in which the selective serotonin/noradrenaline reuptake inhibitor is milnacipran or a salt thereof;

[A20-3] a preparation according to the item [A20-2], in which milnacipran or a salt thereof is milnacipran hydrochloride;

[A21] a method of transdermally administering a selective serotonin/noradrenaline reuptake inhibitor;

[A21-2] a method according to the item [A21], in which the selective serotonin/noradrenaline reuptake inhibitor is milnacipran or a salt thereof;

[A21-3] a method according to the item [A21-2], in which milnacipran or a salt thereof is milnacipran hydrochloride;

[B1] a preparation for transnasal administration, which contains milnacipran or a salt thereof as an active ingredient;

[B2] a preparation for transnasal administration according to the item [B1], in which milnacipran or a salt thereof is milnacipran hydrochloride;

[B3] a preparation for transnasal administration according to the item [B1] or [B2], in which the preparation for transnasal administration is a solution;

[B4] a preparation for transnasal administration according to the item [B1] or [B2], in which the preparation for transnasal administration is a suspension;

[B5] a preparation for transnasal administration according to the item [B1] or [B2], in which the preparation for transnasal administration is an emulsion;

[B6] a preparation for transnasal administration according to any one of the items [B3] to [B5] above, in which an osmotic pressure ratio is about 1 or less;

[B7] a preparation for transnasal administration according to any one of the items [B3] to [B6], including one or more preservatives selected from quaternary ammonium salts and parabens;

[B8] a preparation for transnasal administration according to any one of the items [B1] to [B7], characterized in that the preparation for transnasal administration is an aerosol;

[B9] a preparation for transnasal administration according to any one of the items [B1] and [B2], characterized in that the preparation for transnasal administration is a powder;

[B10] a preparation for transnasal administration according to any one of the items [B1] to [B9], in which the bioavailability is 80% or more;

[B11] a preparation for transnasal administration according to any one of the items [B1] to [B10], characterized in that the time-to-maximum blood concentration is 60 minutes or less;

[B12] a preparation for transnasal administration according to any one of the items [B1] to [B11], which is used for a patient who is hard to be administered via an oral route;

[B13] a preparation for transnasal administration according to any one of the items [B1] to [B12], in which the preparation is an antidepressant;

[B 14] a preparation for transnasal administration according to any one of the items [B1] to [B12], in which the preparation is an analgetic;

[B 15] a preparation for transnasal administration according to any one of the items [B1] to [B 14] above, in which the daily dosage of milnacipran or a salt thereof, serving as an active ingredient, is 50 mg or more;

[B16] a method of administering milnacipran or a salt thereof by nasal drop;

[B 17] a use of milnacipran or a salt thereof for manufacturing a preparation for transnasal administration; and

[B18] a preparation for transnasal administration, which contains an antidepressant as an active ingredient.

Effect of the Invention

According to the present invention, it is possible to provide an SNRI-containing preparation for transmucosal administration, in particular, an SNRI-containing preparation for transnasal administration, which has a higher absorbability compared to a conventional SNRI preparation, can express its therapeutic effect rapidly, and can be readily administered. In addition, it is possible to provide a milnacipran-containing preparation for transmucosal administration, in particular, a milnacipran-containing preparation for transnasal administration, which has a higher absorbability compared to a conventional milnacipran preparation, can express its therapeutic effect rapidly, and can be readily administered. It is further possible to provide a preparation for transdermal administration containing a selective serotonin/noradrenaline reuptake inhibitor, in particular, a milnacipran-containing preparation for transdermal administration. According to the present invention, it is possible to administer milnacipran or a salt thereof at a high dosage to a patient who is hard to be administered via an oral route.

Best Mode for carrying out the Invention

In the present description, the term "selective serotonin/noradrenaline reuptake inhibitor (SNRI)" refers to a medicine that selectively inhibits reuptake of both of serotonin and noradrenaline. The SNRI to be used in the present invention is not particularly limited as long as it is a compound that selectively inhibits reuptake of both of serotonin and noradrenaline, and specific examples thereof include venlafaxine, duloxetine, and milnacipran, more preferably include milnacipran.

In the present invention, the term "venlafaxine" refers to a compound with a chemical name of ($\pm$)-1-[2-(dimethylamino)-1-(4-methoxyphenyl)ethyl]cyclohexanol or, in some cases, a suitable salt thereof, which can be synthesized by a known method (for example, US 4,535,186, Merck Index 12th Edition, Entry 10079).

In the present invention, the term "duloxetine" refers to a compound with a chemical name of (S)-N-methyl-$\gamma$-(1-naphthalenyloxy)-2-thiophenepropanamine or, in some cases, a suitable salt thereof, which can be synthesized by a known method (for example, US 5,023,269, Merck Index 12th Edition, Entry 3518).

In the present invention, the term "milnacipran" refers to a compound with a chemical name of cis-($\pm$)-2-(aminomethyl)-N,N-diethyl-1-phenyl-cyclopropanecarboxyamide, which is also referred to as F2207, TN-912, dalcipran, midalcipran, or midalipran.

Milnacipran or a suitable salt thereof may be used, and milnacipran can be synthesized by a known method (for example, US 4,478,836, Merck Index 12th Edition, Entry 6281).

In the present invention, the SNRI is preferably provided as a free SNRI or a pharmaceutically acceptable salt thereof. The pharmaceutically acceptable salt is not particularly limited as long as it is a salt formed of pharmaceutically acceptable acidic substance and an SNRI, and examples thereof include a salt with an acidic substance to be used for forming a salt of milnacipran described below, preferably include a hydrochloride.

The salt of milnacipran is preferably a pharmaceutically acceptable salt and is not particularly limited as long as it is a salt formed of a pharmaceutically acceptable acidic substance and an SNRI, and examples thereof include a hydrochloride, hydrobromide, nitrate, sulfate, hydrogen sulfate, phosphate, acetate, lactate, succinate, citrate, maleate, tartrate, fumarate, methanesulfonate, p-toluenesulfonate, camphor sulfonate, and mandelate. Of those, preferable is the hydrochloride, i.e., milnacipran hydrochloride, which is also referred to as Toledomin or IXEL.

Note that, in the present description, the term "milnacipran-containing preparation" refers to "a preparation containing milnacipran or a salt thereof".

Milnacipran is a commercially available antidepressant, and it is well-tolerated and is a safer medicine compared to other antidepressants. In the case where the preparation of the present invention is administered to a patient, the daily dosage may be appropriately determined in view of a subject to be treated, severity of the pain, and judgment of a prescribing physician, and the upper limit is preferably 400 mg or less, more preferably 200 mg or less, and still more preferably 150 mg or less in terms of milnacipran hydrochloride serving as an active ingredient. The lower limit is not limited as long as it is the minimum amount that provides the effectiveness of milnacipran as a medicine such as an antidepressant or analgesic, and it is preferably 15 mg or more, more preferably 25 mg or more, still more preferably 30 mg or more, particularly preferably 50 mg or more, and most preferably 100 mg or more. Meanwhile, according to another aspect, the lower limit is preferably 1 mg or more, more preferably 5 mg or more, still more preferably 10 mg or more, and still more preferably 20 mg or more.

In the case of administration at a high dose, the lower limit is preferably 50 mg or more, and more preferably 75 mg or more, still more preferably 100 mg or more, particularly preferably 125 mg or more in terms of milnacipran hydrochloride serving as an active ingredient, while the upper limit may be the same as the above-mentioned upper limit of the daily dosage in terms of milnacipran hydrochloride but is not limited to the range.

Administration may be performed at the above-mentioned dosage a day in once or in several times.

The SNRI-containing preparation of the present invention may be used as a preparation for transdermal administration or a preparation for transmucosal administration, and it is preferably used as a preparation for transmucosal administration. Of those, it is still more preferably used as a preparation for transnasal administration.

Examples of the mucosa in the preparation for transmucosal administration include, but are not limited to, buccal mucosa, oral mucosa, gingival mucosa, nasal mucosa, eye mucosa, ear mucosa, pulmonary mucosa, gastric mucosa, intestinal mucosa, and endometrium, preferably include oral mucosa, gingival mucosa, eye mucosa, nasal mucosa, and pulmonary mucosa, more preferably include eye mucosa and nasal mucosa. Of those, the nasal mucosa is particularly preferable, and the preparation can be used as a preparation for transnasal administration.

The preparation containing milnacipran or a salt thereof as an active ingredient, which is exemplified as a particularly preferable aspect of the present invention, can be used as a preparation for transmucosal administration. The mucosa includes the above-mentioned examples, and according to a particularly preferable aspect of the present invention, the preparation is used as a preparation for transnasal administration. The milnacipran-containing preparation of the present invention can be used as a preparation for transnasal administration because transnasal administration of the preparation provides higher bioavailability of the active ingredient and a shorter time-to-maximum blood concentration compared to oral administration. The transnasal administration is referred to as intranasal administration, in some cases.

The dosage form of the preparation for transmucosal administration of the present invention is not particularly limited as long as the preparation can be administered transmucosally, and examples thereof include powder, solution, suspension, emulsion, sublingual tablet, sublingual capsule, vaginal tablet, ointment, cream, gel, dermatologic paste, or patch. In production of the preparation, various known pharmaceutically acceptable additives may be incorporated.

For example, a method of delivering the preparation to the pulmonary mucosa include: forming the preparation into a solution or microparticles such as powder; passing the preparation through a sprayer designed to change the particle sizes of the sprayed preparation to smaller; and inhaling the preparation as an oral spray. The preparation may be used for the oral mucosa as an oral mucosal patch or for the eye mucosa as an eyelid patch or an ophthalmic solution. A method of spraying the preparation to the nasal cavity includes, but is not limited to: forming the preparation for transnasal administration into a solution or microparticles such as powder; and passing the preparation through a sprayer designed to change the particle sizes of the sprayed preparation to smaller: or forming the preparation into a cream or an ointment; and applying the preparation to the nasal cavity. Depending on the dosage form or administration site, a specific administration method may be appropriately selected. In addition, examples of drugs suitable for transmucosal administration include drugs described in JP 62-195336 A, JP 03-209327 A, JP 05-22685 B, JP 06-107557 A, JP 07-53671 B, JP 08-183741 A, and the dosage forms of the drugs described in the publications may be appropriately selected.

The preparation for transmucosal administration of the present invention is preferably used as a solution, suspension (suspension formulation), or emulsion (emulsion formulation), more preferably used as a solution. In addition, the preparation can be prepared by adding various additives to an active ingredient.

The solution of the preparation for transmucosal administration of the present invention is preferably prepared by dissolving an active ingredient in a solvent and performing pH adjustment and isotonicity adjustment. That is, the components of the solution preferably include, but are not limited to, at least an active ingredient, solvent, buffering agent, and isotonicity agent.

The suspension can be obtained by: adding a suspending agent or another appropriate additive and purified water or oil to an active ingredient; and suspending them by an appropriate method to uniformize the components. The pH and isotonicity of the suspension is preferably adjusted. That is, the components of the suspension preferably include, but are not limited to, at least an active ingredient, solvent, suspending agent, buffering agent, and isotonicity agent.

The emulsion can be obtained by: adding an emulsifier and purified water to an active ingredient; emulsifying them by an appropriate method to uniformize the components. That is, the components of the emulsion preferably include, but are not limited to, at least an active ingredient, solvent, emulsifier, buffering agent, and isotonicity agent.

Examples of the solvent to be used in a solution, suspension (suspension formulation), or emulsion (emulsion formulation) in the preparation for transmucosal administration of the present invention include water and ethanol, preferably include water. Meanwhile, a mixed solvent of water and ethanol is preferably used. Water is preferably the water, water for injection, purified water, and sterilized purified water listed in the Japanese Pharmacopoeia.

In addition, the suspending agent used for the suspension (suspension formulation) in the preparation for transmucosal administration of the present invention is not limited as long as it is generally used and one kind or two or more kinds of the suspending agents may be contained in the suspension (suspension formulation). Examples of the suspending agent include acacia, powdered acacia, sodium alginate, carrageenan, carboxyvinyl polymer, carmellose sodium, powdered agar, glycerin, crystalline cellulose, tragacanth, powdered tragacanth, hydroxypropyl cellulose, propylene glycol, benzyl alcohol, povidone, polyoxyethylene hydrogenated castor oil, polyoxyethylene hydrogenated castor oil 60, polysorbate 80, macrogol 4000, macrogol 6000, olive oil, sesame oil, soy-bean oil, cottonseed oil, peanut oil, and liquid paraffin, and more preferable examples include acacia, powdered acacia, sodium alginate, carrageenan, carmellose sodium, glycerin, crystalline cellulose, tragacanth, powdered tragacanth, hydroxypropyl cellulose, povidone, polyoxyethylene hydrogenated castor oil, polyoxyethylene hydrogenated castor oil 60, macrogol 4000, macrogol 6000, olive oil, sesame oil, soy-bean oil, cottonseed oil, and peanut oil. Still more preferable examples include olive oil, sesame oil, soy-bean oil, cottonseed oil, and peanut oil. In addition, there is another embodiment in which carboxyvinyl polymer, carmellose sodium, powdered agar, glycerin, crystalline cellulose, hydroxypropyl cellulose, propylene glycol, benzyl alcohol, povidone, polysorbate 80, or macrogol 4000 is still more preferred.

The emulsifier used for the emulsion (emulsion formulation) in the preparation for transmucosal administration of the present invention is not limited as long as it is generally used and one kind or two or more kinds of the emulsifiers may be contained in the emulsion (emulsion formulation). Examples of the emulsifier include carboxyvinyl polymer, carmellose sodium, highly purified yolk lecithin, glycerin, hydrogenated soy-bean phospholipid, squalane, squalene, polyoxyl 45 stearate, stearic acid, polyoxyl 55 stearate, purified soy-bean lecithin, purified yolk lecithin, sorbitan sesquioleate, sorbitan esters of fatty acid, soy-bean lecithin, hydroxypropyl cellulose, partially hydrogenated soy-bean phospholipid, propylene glycol, polyoxyethylene hydrogenated castor oil, polyoxyethylene hydrogenated castor oil 5, polyoxyethylene hydrogenated castor oil 10, polyoxyethylene hydrogenated castor oil 20, polyoxyethylene hydrogenated castor oil 40, polyoxyethylene hydrogenated castor oil 50, polyoxyethylene hydrogenated castor oil 60, polyoxyethylene castor oil, polyoxyethylene behenyl ether, polyoxyethylene(160)polyoxypropylene(30) glycol, polyoxyethylene(1)polyoxypropylene(1)cetyl ether, polyoxyethylene(10)polyoxypropylene(4)cetyl ether, polyoxyethylene(20)polyoxypropylene(4)cetyl ether, polyoxyethylene(20)polyoxypropylene(8)cetyl ether, polysorbate 80, macrogol 400, cottonseed oil·soy-bean oil mixture, and sorbitan monostearate. Preferable examples include highly purified yolk lecithin, hydrogenated soy-bean phospholipid, squalane, squalene, polyoxyl 45 stearate, polyoxyl 55 stearate, purified soy-bean lecithin, purified yolk lecithin, sorbitan sesquioleate, sorbitan esters of fatty acid, soy-bean lecithin, partially hydrogenated soy-bean phospholipid, polyoxyethylene hydrogenated castor oil, polyoxyethylene hydrogenated castor oil 5, polyoxyethylene hydrogenated castor oil 10, polyoxyethylene hydrogenated castor oil 20, polyoxyethylene hydrogenated castor oil 40, polyoxyethylene hydrogenated castor oil 50, polyoxyethylene hydrogenated castor oil 60, polyoxyethylene castor oil, polyoxyethylene behenyl ether, polyoxyethylene(160)polyoxypropylene(30) glycol, polyoxyethylene(1)polyoxypropylene (1)cetyl ether, polyoxyethylene(10)polyoxypropylene(4)cetyl ether, polyoxyethylene(20)polyoxypropylene(4)cetyl ether, polyoxyethylene(20)polyoxypropylene(8)cetyl ether, and sorbitan monostearate. In addition, there is another embodiment in which carboxyvinyl polymer, carmellose sodium, glycerin, stearic acid, hydroxypropyl cellulose, propylene glycol, polysorbate 80, macrogol 400, or cottonseed oil·soy-bean oil mixture is preferred.

In the preparation for transmucosal administration of the present invention, pH of exemplified liquid formulation used for the solution, the suspension, and the emulsion having an upper limit of preferably 10 or less, more preferably 9 or less, still more preferably 8 or less, and particularly preferably 7 or less, and having a lower limit of preferably 2 or more, more preferably 3 or more, still more preferably 4 or more, and particularly preferably 5 or more. There is another embodiment in which the upper limit of the pH is preferably 8.5 or less, more preferably 7.5 or less, and still more preferably 6.5 or less, and the lower limit of the pH is preferable 3.8 or more, more preferably 4.3 or more, still more preferably 4.8 or more, particularly preferably 5.3 or more, and most preferably 5.5 or more.

The pH can be adjusted with adding amount of a buffering agent. The buffering agent is not limited as long as it is generally used, and one kind or two or more kinds of the buffering agent may be contained in the solution, the suspending, or the emalsion. Examples of the buffering agent include adipic acid, ammonia water, hydrochloric acid, dried sodium carbonate, diluted hydrochloric acid, citric acid, sodium citrate, monobasic sodium citrate, glycin, glucono-γ-lactone, gluconic acid, sodium dihydrogen phosphate dihydrate, succinic acid, acetic acid, ammonium acetate, sodium acetate, diisopropanol amine, tartaric acid, D-tartaric acid, Sodium L-tartarate, potassium hydroxide, calcium hydroxide, sodium hydroxide, magnesium hydroxide, sodium bicarbonate, sodium carbonate, triisopropanol amine, triethanol amine, lactic acid, calcium lactate, sodium lactate, glacial acetic acid, monosodium fumarate, sodium propionate, boric acid, ammonium pentaborate, borax, maleic acid, anhydrous citric acid, sodium acetic anhydrous, didibasic sodium phosphate anhydrous, disodium dihydrogen phosphate anhydrous, meglumine, methansulfonic acid, monoethanol amine, sulfuric acid, aluminum potassium sulfate, DL-malic acid, phosphoric acid, trisodium phosphate, dibasic sodium phosphate, dibasic potassium phosphate, monobasic potassium phosphate, and sodium hydrogen phosphate dihydrate. Preferable examples include hydrochloric acid, dried sodium carbonate, diluted hydrochloric acid, citric acid, sodium citrate, monobasic sodium citrate, glycin, sodium dihydrogen phosphate dihydrate, succinic acid, acetic acid, ammonium acetate, sodium acetate, tartaric acid, D-tartaric acid, Sodium L-tartarate, potassium hydroxide, calcium hydroxide, sodium hydroxide, magnesium hydroxide, sodium bicarbonate, sodium carbonate, lactic acid, calcium lactate, sodium lactate, glacial acetic acid, monosodium fumarate, sodium propionate, boric acid, ammonium pentaborate, borax, anhydrous citric acid, sodium acetic anhydrous, monohydric sodium phosphoric anhydride, disodium dihydrogen phosphate anhydrous, methansulfonic acid, sulfuric acid, aluminum potassium sulfate, DL-malic acid, phosphoric acid, trisodium phosphate, dibasic sodium phosphate, dibasic potassium phosphate, monobasic potassium phosphate, and sodium hydrogen phosphate dihydrate. More preferable examples include hydrochloric acid, citric acid, sodium citrate, monobasic sodium citrate, sodium dihydrogen phosphate dihydrate, sodium acetate, sodium hydroxide, sodium propionate, boric acid, borax, anhydrous citric acid, disodium dihydrogen phosphate anhydrous, dibasic sodium phosphate, dibasic potassium phosphate, monobasic potassium phosphate, and sodium hydrogen phosphate dihydrate.

The osmotic pressure ratio of the solution, the suspension, and the emalsion of the present invention has an upper limit of preferably 1.5 or less, more preferably 1.4 or less, still more preferably 1.3 or less, particularly preferably 1.2 or less, and most preferably 1.1 or less. The osmotic pressure has a lower limit of preferably 0.5 ore more, more preferably 0.6 or more, still more preferably 0.7 or more, particularly preferably 0.8 or more, and most preferably 0.9 or more. There is another embodiment in which the osmotic pressure ratio of the solution, the suspension, and the emalsion of the present invention has an upper limit of preferably 5.0 or less, more preferably 4.0 or less, still more preferably 3.0 or less, particularly preferably 2.5 or less, and most preferably 2.0 or less, and an lower limit of 0.85 or more, more preferably 0.95 or more, still more preferably 1.05 or more, particularly preferably 1.15 or more, and most preferably 1.25 or more. In addition, there is still another embodiment in which the osmotic pressure is preferably around 1.

The osmotic pressure can be measured by determining the osmolar concentration of a sample by freezing point depression, specifically, by the osmotic pressure measurement method described in the Japanese Pharmacopoeia (14th Edition).

The osmotic pressure ratio can be adjusted with adding amount of a isotonicity agent. The isotonicity agent is not limited as long as it is generally used and one kind or two or more kinds of the isotonicity agents may be contained in the solution, the suspending, and the emalsion. Examples of the isotonicity agent include aminoethyl sulfonic acid, sodium bisulfite, calcium chloride, potassium chloride, sodium chloride, benzalkonium chloride, magnesium chloride, fructose, citric acid, sodium citrate, glycerin, sodium dihydrogen phosphate dihydrate, calcium bromide, sodium bromide, sodium hydroxide, isotonic sodium chloride solution, sodium bicarbonate, D-sorbitol solution, nicotinamide, sodium lactate solution, concentrated glycerin, propylene glycol, benzyl alcohol, boric acid, borax, macrogol 4000, sodium pyrophosphate anhydrous, phosphoric acid, dibasic sodium phosphate, sodium hydrogen phosphate dihydrate, and monobasic potassium phosphate. Preferable examples include aminoethyl sulfonic acid, sodium bisulfite, calcium chloride, potassium chloride, sodium chloride, benzalkonium chloride, magnesium chloride, citric acid, sodium citrate, glycerin, sodium dihydrogen phosphate dihydrate, calcium bromide, sodium bromide, sodium hydroxide, isotonic sodium chloride solution, sodium bicarbonate, D-sorbitol solution, nicotinamide, sodium lactate solution, concentrated glycerin, propylene glycol, benzyl alcohol, boric acid, borax, macrogol 4000, sodium pyrophosphate anhydrous, phosphoric acid, dibasic sodium phosphate, sodium hydrogen phosphate dihydrate, and monobasic potassium phosphate. In addition, more preferable examples include calcium chloride, potassium chloride, sodium chloride, magnesium chloride, citric acid, sodium citrate, glycerin, sodium dihydrogen phosphate dihydrate, sodium hydroxide, isotonic sodium chloride solution, sodium bicarbonate, concentrated glycerin, phosphoric acid, dibasic sodium phosphate, sodium hydrogen phosphate dihydrate, and monobasic potassium phosphate.

In addition, there is another embodiment in which sodium bisulfite, calcium chloride, potassium chloride, sodium chloride, benzalkonium chloride, magnesium chloride, fructose, citric acid, sodium citrate, glycerin, sodium dihydrogen phosphate dihydrate, sodium hydroxide, isotonic sodium chloride solution, sodium bicarbonate, D-sorbitol solution, nicotinamide, concentrated glycerin, propylene glycol, benzyl alcohol, boric acid, borax, macrogol 4000, phosphoric acid, dibasic sodium phosphate, sodium hydrogen phosphate dihydrate, or monobasic potassium phosphate is more preferred. There is still another embodiment in which sodium bisulfite, sodium chloride, benzalkonium chloride, fructose, sodium citrate, glycerin, sodium dihydrogen phosphate dihydrate, sodium hydroxide, isotonic sodium chloride solution, D-sorbitol solution, nicotinamide, concentrated glycerin, propylene glycol, benzyl alcohol, borax, macrogol 4000, dibasic sodium phosphate, sodium hydrogen phosphate dihydrate, or monobasic potassium phosphate is still more preferred.

In the case where the preparation for transmucosal administration of the present invention is a liquid formulation such as a solution, suspension, or emulsion, a thickening agent may be added to thicken the solution in view of adherability to the mucosa. The tickening agent is not particularly limited as long as it is generally used and one kind or two or more kinds of the tickening agent may be contained in the solution, the suspension, and the emalsion. Examples of the thickening agent include sodium alginate, propyleneglycol alginate, ethyl cellulose, carboxyvinyl polymer, carmellose sodium, xanthane gum, glycerin, sodium chondoroitin sulfate, D-sorbitol solution, concentrated glycerin, hydroxyethyl cellulose, hydroxyethylmethyl cellulose, hydroxypropyl cellulose, hydroxypropylmethyl cellulose, propylene glycol, povidone, polysorbate 80, polyvinyl alcohol, macrogol 400, macrogol 4000, methyl cellulose, cottonseed oil·soy-bean oil mixture, and poly-L-alginine. Preferable examples include sodium alginate, propyleneglycol alginate, ethyl cellulose, carboxyvinyl polymer, carmellose sodium, xanthane gum, glycerin, sodium chondoroitin sulfate, hydroxyethyl cellulose, hydroxyethylmethyl cellulose, hydroxypropyl cellulose, hydroxypropylmethyl cellulose, povidone, polyvinyl alcohol, macrogol, methyl cellulose, and poly-L-alginine. In addition, there is another embodiment in which carboxyvinyl polymer, carmellose sodium, glycerin, sodium chondoroitin sulfate, D-sorbitol solution, concentrated glycerin, hydroxypropyl cellulose, propylene glycol, povidone, polysorbate 80, macrogol 400, macrogol 4000, mixture of a cottonseed oil and soy-bean oil, or poly-L-alginine is preferred.

In the case where the preparation for transmucosal administration of the present invention is a liquid formulation such as a solution, suspension, or emulsion, an antiseptic agent may be added to stabilize the active ingredient. The antiseptic agent is not particularly limited as long as it is generally used and one kind or two or more kinds of the antiseptic agent may be contained in the solution, the suspension, and the emalsion. Examples of the antiseptic agent include quaternary ammonium salts such as cetylpyridinium chloride, benzalkonium chloride, benzethonium chloride, and benzetonium chloride solution, parabens such as isobutyl p-oxybenzoate, isopropyl p-oxybenzoate, ethyl p-oxybenzoate, butyl p-oxybenzoate, propyl p-oxybenzoate, and methyl p-oxybenzoate, ethanol, disodium edetate, thimerosal, sodium dehydroacetate, phenol, borax, and boric acid. Preferable examples include quaternary ammonium salts such as cetylpyridinium chloride, benzalkonium chloride, benzethonium chloride, and benzethonium chloride solution, parabens such as isobutyl p-oxybenzoate, isopropyl p-oxybenzoate, ethyl p-oxybenzoate, butyl p-oxybenzoate, propyl p-oxybenzoate, and methyl p-oxybenzoate. More preferable examples include quaternary ammonium salts such as cetylpyridinium chloride, benzalkonium chloride, benzethonium chloride, and benzethonium chloride solution. In addition, there is another embodiment in which benzalkonium chloride, ethyl p-oxybenzoate, butyl p-oxybenzoate, propyl p-oxybenzoate, methyl p-oxybenzoate, ethanol, disodium edetate, thimerosal, sodium dehydroacetate, phenol, borax, and boric acid is preferred.

The preparation for transmucosal administration of the present invention may be used as a powder or aerosol. The aerosol is a product manufactured so that the solution, suspension, emulsion, or the like of milnacipran can be sprayed in use by the pressure of a liquefied gas or compressed gas filled in the same container or another container. The aerosol may be manufactured according to the description in the item of aerosols in the Japanese Pharmacopoeia (14th Edition).

The preparation for transmucosal administration of the present invention may be administered to the mucosa by spraying as a liquid formulation such as a liquid, suspension, or emulsion. For example, in the case of spray administration to the nasal cavity, the liquid formulation may be filled in a nasal drop container, a spray container, or a similar container suitable for applying such a liquid formulation to the nasal cavity. The concentration of the liquid formulation is not particularly limited as long as it is suitable for transnasal administration, and the upper limit of the liquid formulation is, for example, 1,000 mg/ml or less, preferably 800 mg/ml or less, more preferably 600 mg/ml or less, still more preferably 400 mg/ml or less, particularly preferably 300 mg/ml or less, and most preferably 250 mg/ml or less in terms of milnacipran hydrochloride, while the lower limit is, for example, 10 mg/ml or more, preferably 25 mg/ml or more, more preferably 50 mg/ml or more, still more preferably 100 mg/ml or more, particularly preferably 125 mg/ml or more, and most preferably 150 mg/ml or more.

The upper limit of the volume of the preparation administered by spray administration is, for example, 1,000 μL or less, preferably 500 μL or less, more preferably 250 μL or less, still more preferably 200 μL or less, particularly preferably 150 μL or less, and most preferably 125 μL or less, while the lower limit is, for example, 10 μL or more, preferably 30 μL or more, more preferably 50 μL or more, still more preferably 60 μL or more, particularly preferably 70 μL or more, and most preferably 75 μL or more.

Spray administration may be performed once, twice, or several times a day. If necessary, the number of spray administration may be appropriately selected.

In the case of transnasal administration of the preparation of the present invention, the preparation may be administered by a single administration via one nasal cavity or via both the nasal cavities to administer a desired amount of the preparation.

The powder of the preparation for transmucosal administration of the present invention can be produced by a general method, and it can be prepared by adding an excipient or the like to an active ingredient. The excipient is not particularly limited as long as it is generally used and one kind or two or more kinds of the excipient may be contained in the preparation for transmucosal administration. Examples of the excipient include carmellose sodium, croscarmellose sodium, crospovidone, magnesium aluminosilicate, calcium silicate, magnesium silicate, light anhydrous silicic acid, crystalline cellulose, synthesized aluminium silicate, synthesized hydrotalcite, wheat starch, rice starch, sucrose ester of fatty acid, sodium carboxymethyl starch, low-substituted hydroxypropyl cellulose, dextran 40, dextrin, natural aluminium silicate, corn starch, silicon dioxide, lactose, hydroxypropyl cellulose, phenacetin, partially pregelatinized starch, and macrogol 4000. Preferable examples include croscarmellose sodium, crospovidone, aluminum magnesium silicate, calcium silicate, magnesium silicate, light anhydrous silicic acid, crystalline cellulose, synthesized aluminium silicate, synthesized hydrotalcite, wheat starch, rice starch, sucrose ester of fatty acid, sodium carboxymethyl starch, low-substituted hydroxypropyl cellulose, dextran 40, dextrin, natural aluminium silicate, corn starch, silicon dioxide, and partially pregelatinized starch. In addition, there is another embodiment in which carmellose sodium, crystalline cellulose, lactose, hydroxypropyl cellulose, or macrogol 4000 is preferred.

In the present invention, the bioavailability (F) (hereinafter, also referred to as absorption ratio (F)) is a percentage of the amount of a drug absorbed to the amount of the drug administered, and in the present invention, it is represented as the total amount of milnacipran or a salt thereof that appears in blood.

The lower limit of the bioavailability (F) of the preparation for transnasal administration of the present invention is preferably 90% or more, more preferably 93% or more, still more preferably 95% or more, particularly preferably 97% or more, and most preferably 99% or more. In another case, the lower limit is preferably 40% or more, more preferably 50% or more, still more preferably 60% or more, particularly preferably 70% or more, and most preferably 80% or more. In another aspect, the lower limit is preferably 1% or more, more preferably 5% or more, still more preferably 10% or more, particularly preferably 20% or more, and most preferably 30% or more. Although the upper limit of F is not particularly limited as long as F is 100% or less, it is preferably 99% or less.

In the present invention, the maximum blood concentration time (Tmax) is a time between administration of a drug and achievement of the maximum blood concentration when the maximum blood concentration is recognized after the administration of a drug and is used for evaluation of the absorption rate of a drug from an administration site.

The maximum blood concentration time (Tmax) of the preparation for transnasal administration of the present invention is preferably 60 minutes or shorter, more preferably 40 minutes or shorter, still more preferably 30 minutes or shorter, particularly preferably 25 minutes or shorter, and most preferably 20 minutes or shorter. The above-mentioned range is very shorter than 120 minutes, which is known data for oral administration to the human, and it is found that a use of milnacipran or a salt thereof by transnasal administration is effective.

The preparation for transmucosal administration of the present invention may include not only the above-mentioned mixture selected depending on the usage but also a compound that can be used in a general preparation for transnasal administration as long as it has no effect on the efficacy of an active ingredient.

The preparation for transmucosal administration of the present invention may be used for a patient administered with a conventional milnacipran for oral administration, and is effective for, in particular, a patient who is hard to be administered via an oral route or a patient requiring high-dose administration. In addition, the preparation of the present invention can be used for preventing or treating suitable clinical conditions to provide the drug efficacy of a major component in the preparation of the present invention. Specifically, the preparation of the present invention can be used for, but are not limited to, a preparation for transmucosal administration, serving as an antidepressant or analgesic. For example, the preparation may be used for treating stress urinary incontinence, fibromyalgia syndrome (FMS), or the like. The preparation is more preferably used as a known antidepressant. In addition, the preparation is preferably used as a medicine having known drug efficacy, and more preferably used as an analgesic (Obata, H. et al., Anesth Analg 2005; 100: 1406-10). Examples of the pain include pain, preferably include chronic pain, neuropathic pain, headache, migraine, tension headache, chronic pelvic pain, myalgia, arthralgia, and fibromyalgia, more preferably include chronic pain, neuropathic pain, and fibromyalgia, and still more preferably include neuropathic pain and fibromyalgia.

The preparation of the present invention may further be used for treating chronic fatigue syndrome (CFS). Also, the preparation of the present invention may be used for treating neurogenic bladder, overactive bladder (OAB), or interstitial cystitis.

The excellent analgesic effect of the preparation of the present invention can be determined by the method described in Obata, H. et al., Anesth Analg 2005; 100: 1406-10, for example.

Meanwhile, the present invention provides a method of administering an SNRI via a mucosal route, in particular, a method of administering an SNRI by nasal drop. The present invention further provides a method of administering milnacipran or a salt thereof by nasal drop. Milnacipran or a salt thereof is transmucosally absorbed at a high efficiency, and administration of the preparation by nasal drop can provide its effect rapidly and enhance the effect.

In the case of a solid preparation, examples of the method of administering milnacipran or a salt thereof by nasal drop include a method including: placing a capsule filled with powder in a single-purpose spray instrument equipped with a needle; passing the needle through the capsule to make minimal pores through the top and bottom of the capsule; flowing air using a rubber bulb to blow the powder to the nasal cavity.

Meanwhile, in the case where the dosage form is a liquid formulation such as a solution, suspension, or emulsion, examples thereof include: putting the liquid formulation into a nasal drop container, spray container, or similar container suitable for applying the liquid formulation to the nasal cavity; and administering the formulation by dropping or spraying to the nasal cavity. The formulation may be administered as an aerosol.

In the case of a semi-solid formulation such as a cream or ointment, a method including filling the preparation in a tube, attaching an applicator to the end of the tube, and administering the preparation directly to the nasal cavity and a method including putting a predetermined amount of the preparation into a device for intranasal insert and administering the preparation to the nasal cavity.

Examples of a patient to be administered with milnacipran or a salt thereof include a patient suffering from the above-mentioned diseases that can be treated with the preparation of the present invention. Specific examples of the patient include a patient suffering from depression or pain. In addition, milnacipran or a salt thereof may be administered to a patient suffering from stress urinary incontinence or fibromyalgia syndrome (FMS). It is more preferably administered to a patient suffering from depression. Examples of the patient preferably include suffering from another disease, more preferably includes a patient suffering from a pain. Examples of the pain include pain, preferably include chronic pain, neuropathic pain, headache, migraine, tension headache, chronic pelvic pain, myalgia, arthralgia, and fibromyalgia, more preferably include chronic pain, neuropathic pain, and fibromyalgia, and still more preferably include neuropathic pain and fibromyalgia.

In addition, the SNRI-containing preparation of the present invention can be used as a preparation for transdermal administration.

The preparation for transdermal administration of the present invention preferably includes an absorption enhancer. The absorption enhancer is not particularly limited as long as it has an absorption-enhancing effect, and examples thereof include alcohols, higher alkanes, higher fatty acids, higher fatty acid esters, terpenes, alkyl sulfates, alkylamine oxides, pyrrolidones, inclusion-forming compounds, bile salts, saponins, and polyalcohols. Examples of the alcohols include ethanol, isopropanol, decanol, and stearyl alcohol. Moreover, the absorption enhancer may be one described in JP 2006-335714 A.

The dosage form of the preparation for transdermal administration of the present invention is not particularly limited as long as a drug can be supplied during a period required for treatment, and examples thereof include patch, ointment, gel, cream, and liquid formulation. In particular, the patch is preferable because it can supply an effective amount of a drug for a long period of time. Examples of the patch include a cataplasm, tape, patch, and plaster. In the case where the preparation of the present invention is a patch, known base, support, and the like described in JP 2006-335714 A may be used as patches.

Note that a preparation having a form common to that of a preparation for transmucosal administration may be prepared in the same way as the above-mentioned method of preparing the preparation for transmucosal administration.

The present invention also provides a method of using milnacipran or a salt thereof for production of a preparation for transmucosal administration or for transdermal administration. In particular, the present invention provides a method of using milnacipran or a salt thereof for production of a preparation for transnasal administration.

Hereinafter, although the present invention will be described specifically by way of Examples, Test Examples; etc., it is not limited thereto.

Examples

[Example 1]

10 g of milnacipran hydrochloride (synthesized with reference to Patent Documents 1 to 3) was dissolved in an appropriate amount of purified water, and the total volume was adjusted to 50 mL with purified water, to thereby yield a transmucosal formulation containing milnacipran hydrochloride at a concentration of 20 mg/mL. The preparation for transmucosal administration has a pH of 4.55 and an osmotic pressure ratio of 3.81.

[Example 2]

3,500 mg of milnacipran hydrochloride (synthesized with reference to Patent Documents 1 to 3) was dissolved in an appropriate amount of purified water, and the total volume was adjusted to 50 mL with purified water, to thereby yield a preparation for transmucosal administration containing milnacipran hydrochloride at a concentration of 70 mg/mL. The preparation for transmucosal administration has a pH of 4.81 and an osmotic pressure ratio of 1.53.

[Example 3]

3,250 mg of milnacipran hydrochloride (synthesized with reference to Patent Documents 1 to 3) was dissolved in an appropriate amount of purified water, and the total volume was adjusted to 50 mL with purified water, to thereby yield a preparation for transmucosal administration containing milnacipran hydrochloride at a concentration of 65 mg/mL. The preparation for transmucosal administration has a pH of 4.81 and an osmotic pressure ratio of 1.43.

[Example 4]

3,000 mg of milnacipran hydrochloride (synthesized with reference to Patent Documents 1 to 3) was dissolved in an appropriate amount of purified water, and the total volume was adjusted to 50 mL with purified water, to thereby yield a preparation for transmucosal administration containing milnacipran hydrochloride at a concentration of 60 mg/mL. The preparation for transmucosal administration has a pH of 4.79 and an osmotic pressure ratio of 1.33.

[Example 5]

2,500 mg of milnacipran hydrochloride (synthesized with reference to Patent Documents 1 to 3) was dissolved in an appropriate amount of purified water, and the total volume was adjusted to 50 mL with purified water, to thereby yield a preparation for transmucosal administration containing milnacipran hydrochloride at a concentration of 50 mg/mL. The preparation for transmucosal administration has a pH of 4.97 and an osmotic pressure ratio of 1.09.

[Example 6]

2,500 mg of milnacipran hydrochloride (synthesized with reference to Patent Documents 1 to 3), 50 mg of hydroxypropylmethylcellulose (from Nippon Soda Co., Ltd.; HPC-L), 2.27 mg of monobasic potassium phosphate (from Wako Pure Chemical Industries, Ltd.; special grade), 0.7 mg of sodium hydroxide (from Wako Pure Chemical Industries, Ltd.; special grade), and 25 mg of methyl parahydroxybenzoate (from Wako Pure Chemical Industries, Ltd.; special grade) were dissolved in purified water, and the total volume was adjusted to 50 mL, to thereby yield a preparation for transmucosal administration containing milnacipran hydrochloride at a concentration of 50 mg/mL. The preparation for transmucosal administration has a pH of 6.22 and an osmotic pressure ratio of 1.13.

[Example 7]

3,500 mg of milnacipran hydrochloride (synthesized with reference to Patent Documents 1 to 3), 50 mg of hydroxypropylmethylcellulose (from Nippon Soda Co., Ltd.; HPC-L), 22.7 mg of monobasic potassium phosphate (from Wako Pure Chemical Industries, Ltd.; special grade), 7 mg of sodium hydroxide (from Wako Pure Chemical Industries, Ltd.; special grade), and 25 mg of methyl parahydroxybenzoate (from Wako Pure Chemical Industries, Ltd.; special grade) were dissolved in purified water, and the total volume was adjusted to 50 mL, to thereby yield a preparation for transmucosal administration containing milnacipran hydrochloride at a concentration of 70 mg/mL. The preparation for transmucosal administration has a pH of 6.85 and an osmotic pressure ratio of 1.57.

[Example 8]

5,000 mg of milnacipran hydrochloride (synthesized with reference to Patent Documents 1 to 3), 50 mg of hydroxypropylmethylcellulose (from Nippon Soda Co., Ltd.; HPC-L), 22.7 mg of monobasic potassium phosphate (from Wako Pure Chemical Industries, Ltd.; special grade), 7.0 mg of sodium hydroxide (from Wako Pure Chemical Industries, Ltd.; special grade), and 25 mg of methyl parahydroxybenzoate (from Wako Pure Chemical Industries, Ltd.; special grade) were dissolved in purified water, and the total volume was adjusted to 50 mL, to thereby yield a preparation for transmucosal administration containing milnacipran hydrochloride at a concentration of 100 mg/mL. The preparation for transmucosal administration has a pH of 5.91 and an osmotic pressure ratio of 2.13.

[Test Example 1] Observation test of blood kinetics of serotonin noradrenaline selective reuptake inhibitor administered intranasally

<Selective serotonin noradrenaline reuptake inhibitor>

Milnacipran hydrochloride

<Reagents>

In this Test Example, the following reagents were used.
Milnacipran hydrochloride: a synthesized product (synthesized with reference to Patent Documents 1 to 3).
Urethane: Sigma-Aldrich Co. (StLouis, MO, USA)
Acetonitrile for HPLC: Kanto Chemical Co., Ltd.
Chloroform for HPLC, 2-propanol, and n-heptane: Wako Pure Chemical Industries, Ltd. Sodium hydroxide, monobasic potassium phosphate ($KH_2PO_4$), 85% phosphoric acid ($H_3PO_4$), and the other reagents: Wako Pure Chemical Industries, Ltd., special grade chemicals

<Experimental animal>

Wistar male rats (200 to 250 g) were purchased from Saitama Experimental Animals Supply Co., Ltd.

<Experimental Example 1> In vivo intranasal administration test of milnacipran hydrochloride

A solution of urethane in isotonic sodium chloride solution (dosage 1 g/kg, 25 w/v%) was administered intraperitoneally to Wistar male rats (n = 3), and the rats were anaesthetized. Each rat was fixed in a face-up position on a fixed base and underwent an operation of the airway and esophagus to adjust the physiological conditions to the same conditions as those of the intravenous administration test, to thereby expose the left and right jugular veins. The above-prepared solution of milnacipran hydrochloride in isotonic sodium chloride solution was administered to the left nasal cavity using a microsyringe, the top of which was attached to a silicon tube (dosage 10 mg/kg, concentration 50 mg/mL (Example 5)). An injection syringe previously treated with heparin was used to collect blood from the right jugular vein over time. The blood was collected in an amount of 0.2 mL per injection, and the blood samples collected were immediately centrifuged at 4°C and 15,000 rpm for 5 minutes, to thereby yield plasmas.

<Experimental Example 2> In vivo intravenous administration test of milnacipran hydrochloride

A solution of urethane in isotonic sodium chloride solution (dosage 1 g/kg, 25 w/v%) was administered intraperitoneally to Wistar male rats (n = 3), and the rats were anaesthetized. Each rat was fixed in a face-up position on a fixed base and underwent an operation of the airway and esophagus, to thereby expose the left and right jugular veins. The above-prepared solution of milnacipran hydrochloride in isotonic sodium chloride solution was administered from the right jugular vein (dosage 20 mg/kg, concentration 20 mg/mL), and an injection syringe previously treated with heparin was used to collect blood from the left jugular vein over time. The blood was collected in an amount of 0.2 mL per injection, and the blood samples collected were immediately centrifuged at 4°C and 15,000 rpm for 5 minutes, to thereby yield plasmas.

<Method of determining amount of milnacipran hydrochloride>

1) Extraction method

To 100 μL of plasma was added 300 μL of NH$_4$Cl (pH 9.5), and the mixture was stirred for 30 seconds. Then, 500 μL of chloroform/2-isopropanol/n-heptane (60/14/26) was added, and the mixture was shaken for 2 minutes and centrifuged (4°C, 15,000 rpm, 5 min).
The chloroform layer (lower layer) obtained by centrifugation was transferred to another microtube, and the solvent was removed with flowing nitrogen. All the solvents were removed, and the extract was dissolved in 100 μL of a buffer used as a mobile phase for HPLC. Then, the solution was dispensed into a glass tube and injected to HPLC in an amount of 30 μL to measure the concentration of milnacipran hydrochloride.

2) HPLC apparatus

The following HPLC apparatus, from Shimadzu Corporation, was used.
Pump: LC-9A
Detector: SPO-6A
System controller: SCL-6B
Auto-injector: SIL-6B
Column oven: CTO-6A
Chromatopack: C-R6A

3) HPLC conditions

Mobile phase: A solution obtained by deaerating a solution containing acetonitrile and 0.05 M phosphate buffer (pH 3.8) at a ratio of 30:70.
Elution: Flow rate 1 mL/min, 30°C
Measurement wavelength: 200 nm

<Data analysis>

The obtained data was analyzed based on the nonlinear least-squares method program (algorithm: Damping Gauss-Newton method). The area under the plasma concentration-time curve (AUC) was calculated from the trapezoid formula. AUC represents an integrated value of the concentrations of plasma milnacipran absorbed and transferred to blood. The cumulative absorption profile of milnacipran hydrochloride was determined by the deconvolution method using kinetic parameters determined from the intravenous administration test as an input function and plasma levels after transnasal administration as an output function.

<Results>

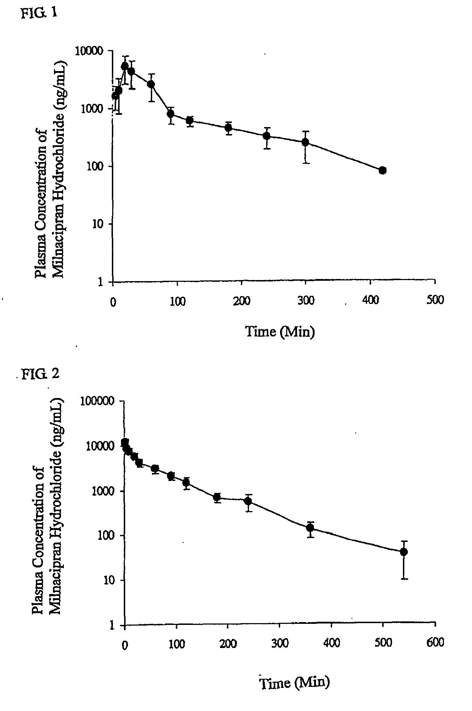

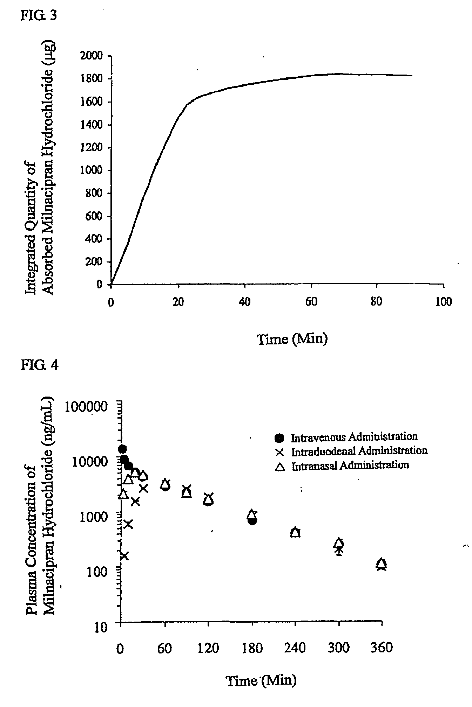

Fig. 1 shows a logarithm of a milnacipran hydrochloride plasma concentration-time curve in the intranasal administration test (Example 1), Fig. 2 shows a logarithm of a milnacipran hydrochloride plasma concentration-time curve in the intravenous administration test (Example 2), and Table 1 shows pharmacokinetic parameters of milnacipran. In addition, Fig. 3 shows the absorption profile of milnacipran hydrochloride, determined by the deconvolution method using kinetic parameters determined from the intravenous administration test and the milnacipran hydrochloride plasma concentration-time curve in the intranasal administration test.
Moreover, Table 2 shows the maximum plasma concentrations (Cmax), time-to-maximum plasma concentrations (Tmax), AUC, and bioavailabilities (F) in intravenous administration (i.v.) and intranasal administration (i.n.).
As shown in Fig. 1 and Table 2, the concentration of milnacipran hydrochloride immediately reached the maximum level after intranasal administration, and Cmax and Tmax levels were found to be 5265.98 ng/mL and 20 min, respectively. Moreover, F is about 100%, which reveals that milnacipran hydrochloride is absorbed well by intranasal administration. The results show that intranasal administration of milnacipran hydrochloride provides a significantly larger absorption rate and a greatly reduced Tmax level compared to the known data for oral administration, i.e., Tmax = about 120 minutes. Moreover, Fig. 3 shows that absorption of milnacipran hydrochloride is very fast and almost completed in about 60 minutes.
Note that the terms "AUC$_{0-7}$" and "F$_{0-7}$" in Table 2 refer to "AUC" and "F" at the time points of 0 to 7 hours, i.e., 0 to 420 minutes, while "AUC$_\infty$" and "F$_\infty$" refer to "AUC" and "F" up to an infinite time. As described above, in the case where provision is made for time of F, F means a value calculated based on AUC up to the time. In the present invention, in the case where no provision is made for time of F, F mainly means a value calculated based on AUC up to an infinite time, in some cases.

[Table 1]

| Table 1 | |
|---|---|
| weight(g) | 238.3 |
| Do(mg) | 4.767 |
| A(ng/mL) | 7085.09 |
| $\alpha$(min$^{-1}$) | 0.1114 |
| $t_{1/2\alpha}$(min) | 6.221 |
| B(ng/mL) | 5580.51 |
| $\beta$(min$^{-1}$) | 0.01079 |
| $t_{1/2\beta}$(min) | 64.252 |
| $k_{21}$(min$^{-1}$) | 0.0551 |
| $k_{10}$(min$^{-1}$) | 0.0218 |
| $k_{12}$(min$^{-1}$) | 0.0453 |
| $V_1$(mL) | 67.74 |
| $V_2$(mL) | 55.64 |
| $V_{55}$(mL) | 123.38 |
| $CL_{tot}$(mL/min) | 2.690 |
| $AUC_{0.7}$(ng·min/mL) | 565566.2 |
| $AUC_\infty$(ng·min/mL) | 580998.4 |

[Table 2]

| | $C_{max}$ (ng/mL) | Tmax (min) | $AUC_{0-7}$ (ng·min/mL) | $AUC_\infty$ (ng·min/mL) | $F_{0-7}$ (%) | $F_\infty$ (%) |
|---|---|---|---|---|---|---|
| i.v.(20 mg/kg) | - | - | 565566.2 | 580998.4 | - | - |
| i.n.(10 mg/kg) | 5265.98 | 20 | 262811.5 | 286429.8 | 90.41 | 95.92 |

[Test Example 2] In vivo mucosal absorption test of selective serotonin noradrenaline reuptake inhibitor

< Selective serotonin noradrenaline reuptake inhibitor>

Milnacipran hydrochloride

<Reagents>

The same reagents as Test Example 1 were used.

<Experimental animal>

Wistar male rats (8 weeks old, 250 to 300 g) were purchased from Saitama Experimental Animals Supply Co., Ltd.

<Experimental Example 3> In vivo intranasal administration experiment of milnacipran hydrochloride

A solution of urethane in isotonic sodium chloride solution (dosage 1 g/kg, 25 w/v%) was administered intraperitoneally to Wistar male rats (n = 4), and the rats were anaesthetized. Each rat was fixed in a face-up position on a fixed base, and the left and right jugular veins were exposed to collect blood. The rats underwent an operation of the airway and esophagus, and the above-prepared isotonic sodium chloride solution containing milnacipran hydrochloride (dosage 20 mg/kg, concentration 200 mg/mL (Example 1)) was administered to the left nasal cavity using a microsyringe, the top of which was attached to a silicon tube. The time of intranasal administration was defined as 0 minutes, and blood samples were collected using an injection syringe previously treated with heparin with time in an amount of 200 μL per injection. The blood samples collected were immediately centrifuged at 4°C and 15,000 rpm (17,860 G) for 5 minutes, to thereby yield plasma samples (100 μL).

<Experimental Example 4> In vivo intravenous administration experiment of milnacipran hydrochloride

A solution of urethane in isotonic sodium chloride solution (dosage 1 g/kg, 25 w/v%) was administered intraperitoneally to Wistar male rats (n = 4), and the rats were anaesthetized. Each rat was fixed in a face-up position on a fixed base, and the left and right jugular veins were exposed to collect blood. The rats underwent an operation of the airway and esophagus to adjust the physiological conditions to the same conditions as those of the intranasal administration test, and the above-prepared isotonic sodium chloride solution containing milnacipran hydrochloride (dosage 20 mg/kg, concentration 20 mg/mL) was administered from the right jugular vein, followed by collection of blood using an injection syringe previously treated with heparin with time in an amount of 200 μL per injection. The blood samples collected were immediately centrifuged at 4°C and 15,000 rpm (17,860 G) for 5 minutes, to thereby yield plasma samples (100 μL).

<Experimental Example 5> In vivo intraduodenal administration experiment of milnacipran hydrochloride

A solution of urethane in isotonic sodium chloride solution (dosage 1 g/kg, 25 w/v%) was administered intraperitoneally to Wistar male rats (n = 4), and the rats were anaesthetized. Each rat was fixed in a face-up position on a fixed base, and the left and right jugular veins were exposed to collect blood. The abdominal cavity was incised to take the duodenum out, and the downstream portion of the duodenum was ligated, followed by direct administration of the above-prepared isotonic sodium chloride solution containing milnacipran hydrochloride (dosage 20 mg/kg, concentration 20 mg/mL) to the duodenum from the upstream portion.
The time of intraduodenal administration was defined as 0 minutes, and blood samples were collected using an injection syringe previously treated with heparin with time in an amount of 200 μL per injection. The blood samples collected were immediately centrifuged at 4°C and 15,000 rpm (17,860 G) for 5 minutes, to thereby yield plasma samples (100 μL).

<Method of determining amount of milnacipran hydrochloride>

1) Extraction method

The same method as Test Example 1 was used.

2) HPLC apparatus

The same apparatus as Test Example 1 was used.

3) HPLC conditions

Mobile phase: A solution obtained by deaerating a solution containing acetonitrile/0.05 M phosphate buffer (pH 2.8) at a ratio of 30:70.
Elution: Flow rate 1 mL/min, 40°C
Measurement wavelength: 200 nm

<Data analysis>

The obtained data was analyzed based on the moment analysis to calculate the area under the plasma concentration-time curve (AUC), mean residence time (MRT), and mean absorption time (MAT). The cumulative absorption profile of milnacipran hydrochloride was calculated by the deconvolution method based on kinetic parameters determined from the intravenous administration test for an input function and based on plasma levels after intraduodenal or intranasal administration for an output function.

<Results>

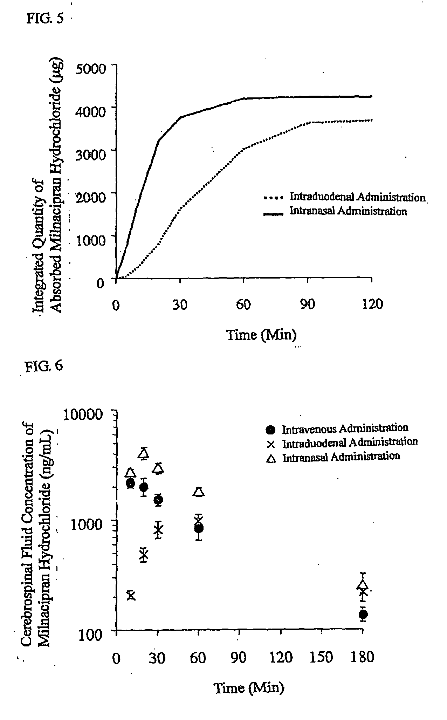

Fig. 4 shows logarithms of plasma levels of milnacipran hydrochloride administered from different administration routes-time curves, and Fig. 5 shows the absorption profile determined by the deconvolution method. In Fig. 4, the symbol "●" shows the result of intravenous administration, the symbol "×" shows the result of intraduodenal administration, and the symbol "Δ" shows the result of intranasal administration. In addition, in Fig. 5, the dashed line shows the result of intraduodenal administration, and the solid line shows the result of intranasal administration. Further, table 3 shows the maximum plasma concentrations (Cmax), time-to-maximum plasma concentrations (Tmax), the area under the plasma concentration-time curve (AUC), and bioavailabilities (F) in intravenous administration (i.v.), intraduodenal administration (i.d.), and intranasal administration (i.n.).

As shown in Fig. 4 and Table 3, Cmax and Tmax levels in intraduodenal administration were found to be 3074.8 ng/mL and 60 minutes, respectively, while Cmax and Tmax levels in intranasal administration were found to be 5124.8 ng/mL and 20 minutes, respectively, which reveals that the concentration of milnacipran hydrochloride immediately reached Cmax by intranasal administration. Moreover, F in intraduodenal administration was found to be 70.8%, while F in intranasal administration was found to be 84.9%, which reveals that milnacipran hydrochloride was absorbed well by intranasal administration. In addition, as shown in Fig. 5, absorption of milnacipran hydrochloride after intranasal administration was very fast and almost completed in about 30 minutes. Meanwhile, MAT in intraduodenal administration was found to be 32.9 minutes, while MAT in intranasal administration was found to be as short as 16.9 minutes, which also reveals that absorption of milnacipran hydrochloride from the nasal mucosa is very fast.

[Table 3]

Table 3

|  | Cmax (ng/mL) | Tmax (min) | $AUC_{\infty}$ (ng·min/mL) | $F_{\infty}$ (%) | $MRT_{\infty}$ (min) | $MAT_{\infty}$ (min) |
| --- | --- | --- | --- | --- | --- | --- |
| i.v.(20 mg/kg) |  | - | 593206.7 | - | 80.3 | - |
| i.d.(20 mg/kg) | 3074.8 | 60 | 420237.4 | 70.8 | 113.2 | 32.9 |
| i.n.(20 mg/kg) | 5124.8 | 20 | 503887.5 | 84.9 | 97.2 | 16.9 |

[Test Example 3] Test for confirming penetration of selective serotonin noradrenaline reuptake inhibitor after intranasal administration to central nerve system

< Selective serotonin noradrenaline reuptake inhibitor>

Milnacipran hydrochloride

<Reagent and experimental animal>

The same reagents and experimental animals as Test Example 2 were used.

< Experimental Example 6> Evaluation experiment of penetration of milnacipran hydrochloride to central nerve system

As an indicator of penetration to the central nerve system, the concentration of a drug in a cerebrospinal fluid (CSF) was measured. The same operations and administrations as Test Example 2 (in vivo mucosal absorption experiment) were performed (n = 3) for intravenous administration, intraduodenal administration, and intranasal administration. Then, blood was collected with time from the jugular vein, and after a given length of time, CSF was collected. An injection needle previously cut was adhered to a silicon tube to prepare an instrument, the other end of which was connected to a syringe, and after a given length of time following drug administration, a puncture was made ventrally from the top of the back of the head of a rat to a depth of 5 to 6 mm with the top of the injection needle. Then, the syringe was drawn to collect CSF in an amount of about 200 μL in the silicon tube. After puncture, in order to confirm contamination of blood into CSF, the erythrocyte content was determined using an optical microscope and was found to be 500 cells/μL or less.

Blood samples collected were immediately centrifuged at 4°C and 15,000 rpm (17,860 G) for 5 minutes, to thereby yield CSF samples (100 μL).

<Method of determining amount of milnacipran hydrochloride>

Determination was performed by the same extraction method, HPLC apparatus, and HPLC conditions as Test Example 2.

<Data analysis>

The obtained data was analyzed based on the moment analysis to calculate the area under the cerebrospinal fluid concentration-time curve ($AUC_{CSF}$). The $AUC_{CSF}$ represents an integrated value of concentrations of milnacipran in CFS, absorbed and transferred to the brain. Penetration of a drug from the general circulation to CSF is controlled by permeation through a blood-brain barrier and is greatly affected by the concentration of the drug in blood. Therefore, in order to consider the effect of permeation through a blood-brain barrier, brain penetration was evaluated by comparing ratios of the concentrations in CSF to the protein unbound drug concentration in plasma at the time of collection of CSF. Meanwhile, the brain penetration ratio ($K_{pu}$) was calculated from an area under the CSF concentration-time curve, an area under the plasma concentration-time curve, and a plasma protein unbinding ratio (Formula 1). A significant test was performed based on the Tukey-Kramer multiple comparison. Note that $AUC_{Plasma}$ represents an integrated value of concentrations of milnacipran in plasmas, absorbed and transferred to the brain.

[Formula 1]

$$K_{pu} = \frac{AUC_{CSF}}{f_t \cdot AUC_{Plasma}} \quad \cdots\cdots(1)$$

<Results>

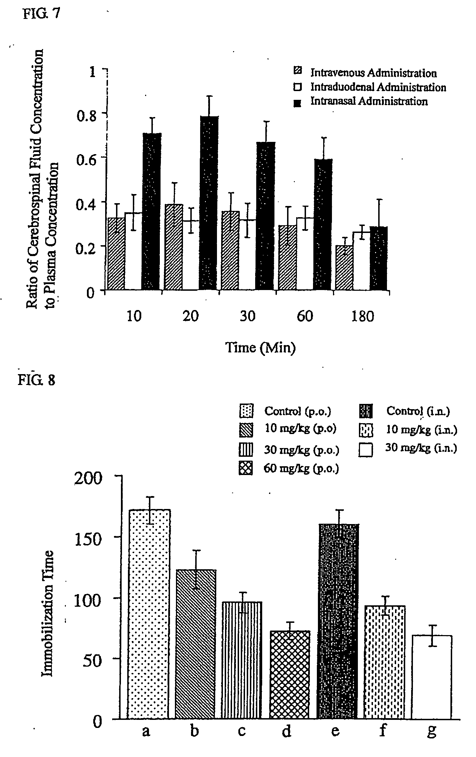

Fig. 6 shows logarithms of CSF levels of milnacipran hydrochloride administered from different administration routes-time curves, Fig. 7 shows ratios of plasma concentrations and CSF concentrations, and Table 4 shows maximum CSF concentrations (Cmax), time-to-maximum CSF concentrations (Tmax), areas under the CSF concentration-time curves ($AUC_{CSF}$), areas under the plasma concentration-time curves ($AUC_{Plasma}$), and brain penetration ratio ($K_{pu}$). In Fig. 6, the symbol "●" shows the result of intravenous administration, the symbol "×" shows the result of intraduodenal administration, and the symbol "Δ" shows the result of intranasal administration. Meanwhile, in Fig. 7, the shaded bars show the results of intravenous administration, the unfilled bars show the results of intraduodenal administration, and the filled bars show the results of intranasal administration. As shown in Table 4, the Cmax levels in intravenous administration and intraduodenal administration were found to be 2216.2 ng/mL and 1005.5 ng/mL, respectively, while the Cmax level in intranasal administration was found to be 4019.1 ng/mL, which was twice larger than that in intravenous administration and was four times larger than that in intraduodenal administration. The Tmax level of intranasal administration was 20 minutes, which reveals that intranasal administration can penetration milnacipran hydrochloride to the brain in a short time compared to the value in intraduodenal administration (60 minutes). In addition, the ratios of plasma concentrations and CSF concentrations in administration routes other than intranasal administration were almost constant, but in the case of intranasal administration, the ratio significantly increased up to 30 minutes after administration.

[Table 4]

Table 4

| | Cmax (ng/mL) | Tmax (min) | $AUC_{CSF}$ (ng·min/mL) | $AUC_{Plasma}$ (ng·min/mL) | $K_{pu}$ |
|---|---|---|---|---|---|
| i.v.(20 mg/kg) | 2216.2 | 10 | 13727.9 | 593206.7 | 0.028 |
| i.d.(20 mg/kg) | 1005.5 | 60 | 8568.1 | 420237.4 | 0.024 |
| i.n.(20 mg/kg) | 4019.1 | 20 | 289425.1 | 503887.5 | 0.684 |

[Test Example 4] Pharmacological evaluation test of selective serotonin noradrenaline reuptake inhibitor after transnasal administration

< Selective serotonin noradrenaline reuptake inhibitor>

Milnacipran hydrochloride

<Reagents>

In this Test Example, the following reagents were used. Milnacipran hydrochloride: a synthesized product (synthesized with reference to Patent Documents 1 to 3)
Diethyl ether: Wako Pure Chemical Industries, Ltd.

<Experimental animal>

The same experimental animals as Test Example 2 were used.

<Tank for swimming>

An acrylic cylindrical tank with a diameter of 18 cm and a height of 40 cm was used.

<Experimental Example 7> Forced swimming test

Wistar male rats purchased were preliminarily fed for 6 days, and a habituation test was performed for 15 minutes in the previous day of the main test, followed by the main test for 5 minutes. A tank for swimming was filled with water heated to 25°C up to a depth of 18 cm. The rats were forced to swim, and changes in the behaviors were observed using a Web camera placed just above the tank to measure immobilization times. After completion of the habituation test and 60 minutes before the start of the main test, the rats were anaesthetized with diethyl ether and administered with isotonic sodium chloride solution containing a selective serotonin noradrenaline reuptake inhibitor (isotonic sodium chloride solution containing milnacipran hydrochloride) from various administration routes (10 mg/kg, 30 mg/kg, and 60 mg/kg for oral administration; 10 mg/kg and 30 mg/kg for transnasal administration). The oral administration was performed using a stomach tube, and the transnasal administration was performed using an instrument produced by connecting a silicon tube to a microsyringe. Meanwhile, isotonic sodium chloride solution was administered as a control agent.

<Statistical analysis>

A significant test was performed based on the Tukey-Kramer multiple comparison.

<Results>

Fig. 8 shows immobilization times in the forced swimming test for rats administered with milnacipran hydrochloride by the following administration routes and dosages: a to g. In Fig. 8, the legend symbols a to g mean the terms in the parentheses.

    a: Oral administration of isotonic sodium chloride solution (Control (p.o.))
    b: Oral administration of 10 mg/kg milnacipran hydrochloride (10 mg/kg (p.o.))
    c: Oral administration of 30 mg/kg milnacipran hydrochloride (30 mg/kg (p.o.))
    d: Oral administration of 60 mg/kg milnacipran hydrochloride (60 mg/kg (p.o.))
    e: Transnasal administration of isotonic sodium chloride solution (Control (i.n.))
    f: Transnasal administration of 10 mg/kg milnacipran hydrochloride (10 mg/kg (i.n.))
    g: Transnasal administration of 30 mg/kg milnacipran hydrochloride (30 mg/kg (i.n.))

Regardless of administration routes, administration of milnacipran hydrochloride provided a significant dose-dependent effect for shortening the immobilization times. Meanwhile, in the cases of the groups administered with isotonic sodium chloride solution as a control agent (a and e), there is no difference between the immobilization time for oral administration and the immobilization time for transnasal administration, and therefore, the difference between the administration routes was considered to have no effect on the times. The oral administration of 30 mg/kg milnacipran hydrochloride (c) and transnasal administration of 10 mg/kg milnacipran hydrochloride (f) provided almost the same effects, while the oral administration of 60 mg/kg milnacipran hydrochloride (c) and transnasal administration of 30 mg/kg milnacipran hydrochloride (f) provided almost the same effects, which reveals that the effect for shortening the immobilization time of the transnasal administration group is stronger than that of the oral administration group.

Industrial Applicability

According to the present invention, it is possible to administer an SNRI transmucosally, in particular, transnasally, to administer an SNRI to a patient who is hard to be orally administered, and to administer at an ideal high dose. In addition, it is possible to administer a milnacipran preparation transmucosally, in particular, transnasally, to administer a milnacipran preparation to a patient who is hard to be orally administered, to administer at an ideal high dose, and to achieve a more effective treatment compared to conventional oral administration of a milnacipran formulation. Moreover, the present invention provides an SNRI-containing transdermal formulation, in particular, a milnacipran-containing transdermal formulation.

Brief Description of the Drawings

[Fig. 1] Fig. 1 is a graph showing a logarithm of a milnacipran hydrochloride plasma concentration-time curve in the intranasal administration test.

[Fig. 2] Fig. 2 is a graph showing a logarithm of a milnacipran hydrochloride plasma concentration-time curve in the intravenous administration test.

[Fig. 3] Fig. 3 is a graph showing the absorption profile of milnacipran hydrochloride in transnasal administration.

[Fig. 4] Fig. 4 is a graph showing logarithms of milnacipran hydrochloride plasma concentration-time curves in intravenous administration, intraduodenal administration, and intranasal administration tests.

[Fig. 5] Fig. 5 is a graph showing the absorption profiles of milnacipran hydrochloride in intraduodenal administration and transnasal administration.

[Fig. 6] Fig. 6 is a graph showing logarithms of milnacipran hydrochloride cerebrospinal fluid concentration-time curves in intravenous administration, intraduodenal administration, and intranasal administration tests.

[Fig. 7] Fig. 7 is a graph showing ratios of the concentrations of milnacipran hydrochloride in cerebrospinal fluids to the concentrations of milnacipran hydrochloride in plasmas.

[Fig. 8] Fig. 8 is a graph showing immobilization times in oral administration and transnasal administration of milnacipran hydrochloride.

**Claims**

1. A preparation for transmucosal administration, comprising a selective serotonin/noradrenaline reuptake inhibitor.

2. A preparation according to Claim 1, wherein the preparation for transmucosal administration comprises a preparation for transnasal administration.

3. A preparation according to Claim 1 or 2, wherein the selective serotonin/noradrenaline reuptake inhibitor is milnacipran or a salt thereof.

4. A preparation according to any one of Claims 1 to 3, wherein the preparation is a solution.

5. A preparation according to any one of Claims 1 to 3, wherein the preparation is a suspension.

6. A preparation according to any one of Claims 1 to 3, wherein the preparation is an emulsion.

7. A preparation according to any one of Claims 4 to 6, wherein an osmotic pressure ratio of the solution, the suspension, or the emulsion is 0.5 to 5.0.

8. A preparation according to any one of Claims 1 to 7, wherein the preparation is an aerosol.

9. A preparation according to any one of Claims 1 to 3, wherein the preparation is a powder.

10. A preparation according to any one of Claims 1 to 9, comprising one or more preservatives selected from quaternary ammonium salts and parabens.

11. A preparation according to any one of Claims 1 to 10, wherein the preparation is an antidepressant.

**12.** A preparation according to any one of Claims 1 to 10, wherein the preparation is an analgetic.

**13.** A preparation for transdermal administration, comprising a selective serotonin/noradrenaline reuptake inhibitor.

**14.** A use of milnacipran or a salt thereof for manufacturing a preparation for transmucosal administration or for transdermal administration containing a selective serotonin/noradrenaline reuptake inhibitor.

**15.** A method of transmucosally or transdermally administering a selective serotonin/noradrenaline reuptake inhibitor.

FIG. 1

FIG. 2

FIG. 3

FIG. 4

FIG. 5

FIG. 6

FIG. 7

FIG. 8

## INTERNATIONAL SEARCH REPORT

| | International application No. |
|---|---|
| | PCT/JP2007/051241 |

**A. CLASSIFICATION OF SUBJECT MATTER**
*A61K45/00*(2006.01)i, *A61K9/08*(2006.01)i, *A61K9/10*(2006.01)i,
*A61K9/107*(2006.01)i, *A61K9/12*(2006.01)i, *A61K9/14*(2006.01)i,
*A61K31/165*(2006.01)i, *A61K47/14*(2006.01)i, *A61K47/18*(2006.01)i,
According to International Patent Classification (IPC) or to both national classification and IPC

**B. FIELDS SEARCHED**

Minimum documentation searched (classification system followed by classification symbols)
A61K45/00, A61K9/08, A61K9/10, A61K9/107, A61K9/12, A61K9/14, A61K31/165,
A61K47/14, A61K47/18, A61P25/24, A61P29/00

Documentation searched other than minimum documentation to the extent that such documents are included in the fields searched
Jitsuyo Shinan Koho          1922-1996    Jitsuyo Shinan Toroku Koho    1996-2007
Kokai Jitsuyo Shinan Koho    1971-2007    Toroku Jitsuyo Shinan Koho    1994-2007

Electronic data base consulted during the international search (name of data base and, where practicable, search terms used)
BIOSIS(STN), CAplus(STN), EMBASE(STN), MEDLINE(STN)

**C. DOCUMENTS CONSIDERED TO BE RELEVANT**

| Category* | Citation of document, with indication, where appropriate, of the relevant passages | Relevant to claim No. |
|---|---|---|
| Y | JP 58-004752 A (Pierre Fabre S.A.), 11 January, 1983 (11.01.83), Example 4; experimental examples & US 4478836 A          & EP 0068999 A1 | 1-14 |
| Y | US 2004/0162334 A1 (JEAN DEREGNAUCOURT), 19 August, 2004 (19.08.04), Full text; particularly, Par. Nos. [0004], [0077], [0100] & JP 2006-517571 A          & WO 2004/075886 A1 | 1-14 |

| ☒ | Further documents are listed in the continuation of Box C. | ☐ | See patent family annex. |
|---|---|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 08 March, 2007 (08.03.07) | 20 March, 2007 (20.03.07) |

| Name and mailing address of the ISA/ | Authorized officer |
|---|---|
| Japanese Patent Office | |
| Facsimile No. | Telephone No. |

Form PCT/ISA/210 (second sheet) (April 2005)

**INTERNATIONAL SEARCH REPORT**

International application No.

PCT/JP2007/051241

C (Continuation).   DOCUMENTS CONSIDERED TO BE RELEVANT

| Category* | Citation of document, with indication, where appropriate, of the relevant passages | Relevant to claim No. |
|---|---|---|
| Y | JP 2005-532372 A  (Nastech Pharmaceutical Company Inc.), 27 October, 2005 (27.10.05), Full text; particularly, Par. Nos. [0012], [0013], [0029] & EP 1505971 A2        & WO 2004/002402 A2 & US 2003/0225031 A1 | 1-14 |
| Y | JP 2001-131057 A  (Takeda Chemical Industries, Ltd.), 15 May, 2001 (15.05.01), Full text; particularly, Par. Nos. [0017], [0044] & EP 1206943 A1        & WO 2001/015735 A1 & US 6663883 B | 1-14 |

Form PCT/ISA/210 (continuation of second sheet) (April 2005)

**INTERNATIONAL SEARCH REPORT**

International application No.

PCT/JP2007/051241

Continuation of A. CLASSIFICATION OF SUBJECT MATTER
(International Patent Classification (IPC))

*A61P25/24*(2006.01)i, *A61P29/00*(2006.01)i

(According to International Patent Classification (IPC) or to both national classification and IPC)

Form PCT/ISA/210 (extra sheet) (April 2005)

## INTERNATIONAL SEARCH REPORT

| International application No. |
|---|
| PCT/JP2007/051241 |

**Box No. II      Observations where certain claims were found unsearchable (Continuation of item 2 of first sheet)**

This international search report has not been established in respect of certain claims under Article 17(2)(a) for the following reasons:

1. ☒ Claims Nos.: 15
   because they relate to subject matter not required to be searched by this Authority, namely:
   Claim 15 pertains to methods for treatment of the human body by therapy and thus relates to a subject matter which this International Searching Authority is not required, under the provisions of the PCT Rule 39.1(iv), to search.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box No. III      Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant.   Consequently, this international search report is restricted to the invention first mentioned in the claims; it is covered by claims Nos.:

**Remark on Protest**
the

☐ The additional search fees were accompanied by the applicant's protest and, where applicable, payment of a protest fee..

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (April 2005)

**INTERNATIONAL SEARCH REPORT**

International application No.

PCT/JP2007/051241

```
<Subject of search>

    Claims 1 and 13 relate to a preparation for transmucosal administration
comprising a substance that is defined by a desired property "selective
serotonin-noradrenaline reuptake inhibitor".
    Claims 1 and 13 include any substance having the property.  However,
those substances which are disclosed in the meaning within PCT Article
5 are limited to an extremely small part of the claimed substances.
Therefore, it is considered that these claims are not supported by the
disclosure of the description in the meaning within PCT Article 6.
    With respect to the property "selective serotonin-noradrenaline
reuptake inhibitor", even though the common technical knowledge at the
time of filing the present application is taken into the consideration,
it appears that the scope of the compound having the property cannot
be specified.  Thus, claims 1 and 13 do not comply with the requirement
of clearness under PCT Article 6, too.
    Such being the case, a search was made on the relationship between
the selective serotonin-noradrenaline reuptake inhibition and the
transmucosal administration and also made on the preparation for
transmucosal administration comprising a substance that is specifically
recited in the description and defined in claims 3 and 14.
```

Form PCT/ISA/210 (extra sheet) (April 2005)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 63023186 B
- JP 5067136 B
- JP 2964041 B
- JP 2000516946 A
- JP 2002519370 A
- US 4535186 A
- US 5023269 A
- US 4478836 A

- JP 62195336 A
- JP 3209327 A
- JP 5022685 B
- JP 6107557 A
- JP 7053671 B
- JP 8183741 A
- JP 2006335714 A

**Non-patent literature cited in the description**

- *Psychopharmacology,* 2002, vol. 5, 93-99
- **OBATA, H. et al.** *Anesth Analg,* 2005, vol. 100, 1406-10